# EUROPEAN PATENT APPLICATION

(11) **EP 3 900 768 A1**
(43) Date of publication of application: **27.10.2021**
(21) Application number: 19898329.8
(22) Date of filing: 19.12.2019
(51) Int. Cl.: A61M 25/00, A61F 7/12, A61B 18/00

(54) **ESOPHAGEAL LIQUID-SUPPLY CATHETER, AND INTERMEDIATE COMPONENT OF ESOPHAGEAL LIQUID-SUPPLY CATHETER**

(30) Priority: 20.12.2018 JP 2018238594
(71) Applicant: Toray Industries, Inc., Tokyo, 103-8666 (JP)
(72) Inventor: YAMAMOTO, Masahiro, Otsu-shi, Shiga 520-2141 (JP); NAKAJIMA, Hiroki, Otsu-shi, Shiga 520-2141 (JP); IKEDA, Tomohiko, Tokyo 103-8666 (JP); MATSUKUMA, Akinori, Otsu-shi, Shiga 520-2141 (JP); OKADA, Tatsuya, Otsu-shi, Shiga 520-2141 (JP)
(74) Representative: Kador & Partner PartG mbB
(86) International application number: PCT/JP2019/049815
(87) International publication number: WO 2020/130074

(57) **Abstract**

An esophageal liquid-supply catheter which is capable of reducing the temperature transmission to the esophagus due to heating, cooling or the like by ablation, by efficiently delivering a liquid to the esophagus is disclosed. The esophageal liquid-supply catheter contains a tubular member, a plurality of arms with the proximal ends fixed to the distal end of the tubular member, and a distal end tip fixed to the distal end of the arms, wherein the plurality of arms can be deformed and expanded outward in the radial direction of the tubular member. For example, a shaft which is inserted through the tubular member and which is fixed on the distal end to the distal end tip is provided, and the arms can be expanded outward in the radial direction when the shaft is slid toward the proximal end side relative to the tubular member.

## Description

### TECHNICAL FIELD

The present invention relates to an esophageal liquid-supply catheter, and an intermediate component for producing the esophageal liquid-supply catheter.

### BACKGROUND ART

Catheter ablation is a method that treats arrhythmias or the like, such as atrial fibrillation, in which a catheter is inserted into the body and the tip of the catheter is heated or cooled to destroy and treat the target site in the heart.

Various catheters have been developed as ablation catheters used in this treatment method. Examples thereof include an ablation catheter with a balloon which is attached at the tip of the catheter and which can be heated in the atria, an ablation catheter with a balloon which is capable of cryoablation, and the like.

When ablation of the heart is performed in this way. the effect of ablation (temperature) may be transmitted to the esophagus close to the heart, causing esophageal complications. Therefore, various measures have been taken.

Patent Document 1 describes a feeding catheter. Not limited to this catheter, similar catheter tubes may be used to deliver a liquid to the esophagus to reduce the temperature transmission to the esophagus.

Patent Document 2 discloses a device that keeps the position of the esophagus away from the ablation site in order to reduce the temperature transmission to the esophagus.

Patent Document 3 describes a device that inserts a balloon into the esophagus and circulates a temperature-controlled liquid in the balloon.

### PRIOR ART DOCUMENTS

### PATENT DOCUMENTS

Patent Document 1: JP 2011-078456 A
Patent Document 2: JP 2016-067728 A
Patent Document 3: JP 2017-225791 A

### SUMMARY OF THE INVENTION

### PROBLEMS TO BE SOLVED BY THE INVENTION

However, when a liquid is delivered to the esophagus using a catheter tube represented by Patent Document 1, it is possible that an efficient liquid delivery cannot be achieved if the esophagus is pressed and compressed from the outside due to the patient's condition, the weight of an organ, or the like.

Further, even if the position of the esophagus is displaced as in Patent Document 2, there is a possibility that the temperature transmission cannot be completely prevented.

In Patent Document 3, the temperature is adjusted by contacting the balloon in which a liquid is circulated with the esophagus, but since the temperature is adjusted only at the contact site of the balloon, it is possible that the reduction of the temperature transmission is less efficient than the method in which the liquid is directly delivered into the esophagus.

An object of the present invention is to reduce the temperature transmission to the esophagus due to heating, cooling or the like by ablation by efficiently delivering a liquid to the esophagus.

### SOLUTIONS TO THE PROBLEMS

As a result of intensive research to solve the above problems, the present inventors have discovered that, by providing a liquid-supply catheter with a plurality of arms near the distal end thereof, in which the arms can be deformed in the esophagus and expanded outward in the radial direction of the catheter, and delivering a liquid when the arms are expanded, it is possible to reduce the temperature transmission to the esophagus due to heating, cooling or the like by ablation, resulting in the present invention.

That is, the present invention provides the following:

(1) An esophageal liquid-supply catheter, comprising a tubular member, a plurality of arms with the proximal ends fixed to the distal end of the tubular member, and a distal end tip fixed to the distal ends of the arms, wherein the plurality of arms can be deformed and expanded outward in the radial direction of the tubular member.
(2) The esophageal liquid-supply catheter according to (1), comprising a shaft which is inserted through the tubular member and which is fixed on the distal end to the distal end tip, wherein the arms are expanded outward in the radial direction when the shaft is slid toward the proximal end relative to the tubular member.
(3) The esophageal liquid-supply catheter according to (2), wherein a space exists between the inner wall of the tubular member and the outer wall of the shaft, and a liquid is flowed in the space.
(4) The esophageal liquid-supply catheter according to (1) or (2), further comprising a liquid-supply tube on the outer wall of the tubular member.
(5) The esophageal liquid-supply catheter according to (2), wherein the shaft is in the form of a catheter, and a liquid to be delivered is flowed through the shaft.
(6) The esophageal liquid-supply catheter according to any one of (1) to (5), wherein the arms or the shaft has a temperature sensor.
(7) The esophageal liquid-supply catheter according to any one of (1) to (6), wherein the tubular member has two or more slits, and the arms are constituted by the tubular member present between the slits.
(8) The esophageal liquid-supply catheter according to any one of (1) to (7), wherein the cross section of the arms has a maximum width of 1 mm or more.
(9) The esophageal liquid-supply catheter according to any one of (2) to (8), wherein the distal end tip and the distal end of the shaft can be fitted with and separated from each other.
(10) The esophageal liquid-supply catheter according to any one of (2), (5) to (9), wherein the shaft is a catheter having a diameter of 1.667 mm to 5 mm.
(11) An intermediate component for producing the esophageal liquid-supply catheter according to any one of (2) to (9), wherein the shaft is excluded from the esophageal liquid-supply catheter comprising the shaft according to any one of (2) to (9).

### EFFECTS OF THE INVENTION

According to the present invention, the arms can be expanded in the esophagus, and by delivering a liquid in this state, it is possible to reduce the temperature transmission to the esophagus due to heating, cooling or the like by ablation.

### BRIEF DESCRIPTION OF THE DRAWINGS

Figure 1 is a schematic view of an esophageal liquid-supply catheter of the first embodiment.
Figure 2 is a schematic view illustrating examples of the cross-sectional shape of the arms.
Figure 3 is a schematic view of an esophageal liquid-supply catheter of the second embodiment.
Figure 4 is a schematic view of an esophageal liquid-supply catheter of the third embodiment.
Figure 5 is a schematic view of an esophageal liquid-supply catheter of the fourth embodiment. Figure 5A shows the state in which the arms are closed, and Figure 5B shows the state in which the arms are expanded outward in the radial direction.
Figure 6 is a schematic view of an esophageal liquid-supply catheter of the fifth embodiment.
Figure 7 is a schematic view of an esophageal liquid-supply catheter of Comparative Example 1.
Figure 8 is a schematic view illustrating an evaluation system for confirming the reduction effect of temperature transmission by an esophageal liquid-supply catheter.

### MODE FOR CARRYING OUT THE INVENTION

Suitable embodiments of the present invention will be described in detail with reference to Figures, but the present invention is not limited to these embodiments. The same elements are denoted by the same reference numerals and redundant description will be omitted. Also, the proportions in Figures do not always match what is described. The term "length" refers to the length in the longitudinal direction.

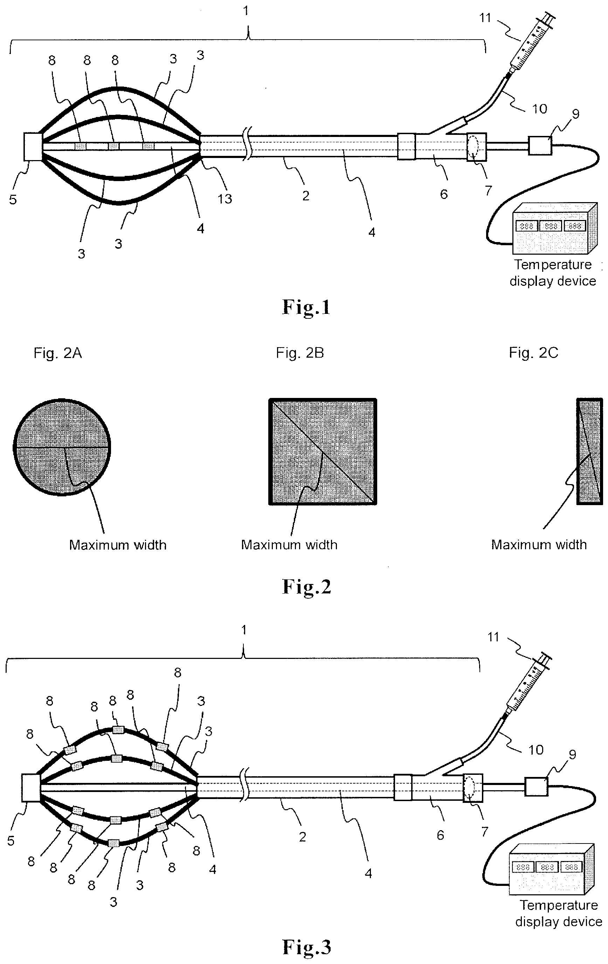

An esophageal liquid-supply catheter 1 of the first embodiment of the present invention will be described with reference to Figure 1.

The esophageal liquid-supply catheter 1 has a tubular member 2 and a shaft 4 inserted into the lumen (space) of the tubular member 2, and the tubular member 2 and the shaft 4 can slide with each other. A space exists between the inner wall of the tubular member 2 and the outer wall of the shaft 4, and a liquid is flowed in the space during the operation.

The examples of the shape of the shaft 4 include, but are not limited to, a tube having a lumen, a wire, a stranded wire, and the like.

The length of the tubular member 2 and the shaft 4 are preferably 200 mm to 1000 mm, respectively. The material of the tubular member 2 and the shaft 4 is preferably a flexible material having excellent antithrombotic properties, and examples thereof include, but are not limited to, fluoropolymers, polyamides, polyurethane polymers, polyimides, metals, and the like.

The tubular member 2 has on its distal end a plurality of arms 3 which are connected to the tubular member 2, and the distal end side of the arms 3 and the distal end side of the shaft 4 are fixed to a distal end tip 5. When the shaft 4 is slid toward the proximal end relative to the tubular member 2, that is, in the direction in which the distal end tip 5 approaches the tubular member 2, the distal end tip 5 and the distal end side of the arms 3 are pulled by the shaft 4 towards the proximal end side. On the other hand, the proximal end side of the arms 3 is fixed to the tubular member 2 and thus does not move. Therefore, as shown in the figure, by the sliding operation of the shaft 4 and the tubular member 2, the arms 3 are deformed and expanded outward in the radial direction. In the case of the insertion through the opening of a person's mouth or nose, the expansion is manipulated as necessary, for example, the arms 3 are inserted in a non-expanded state, and then the arms 3 are expanded outward in the radial direction at the target site. Figure 1 shows a state in which the arms 3 are expanded.

The length of the arms 3 is preferably 20 mm to 100 mm. The cross-sectional shape of the arms 3 may be any shape such as a circle shown in Figure 2A, a square shown in Figure 2B, and a rectangle shown in Figure 2C. Further, the maximum width of the cross section of the arms 3 is preferably 1 mm or more. The upper limit of the maximum width is not particularly limited, but is usually 10 mm or less, particularly 5 mm or less. The maximum width of the cross section is the distance between two most distant points on the cross section. As shown in Figures 2A, 2B and 2C, for example, when the cross-sectional shape of the arms 3 is a circle, the maximum width is the diameter. When the arms 3 have a shape of a square or a rectangle, the maximum width is the diagonal length.

The material of the arms 3 and the distal end tip 5 is preferably a flexible material having excellent antithrombotic properties, and examples thereof include, but are not limited to, fluoropolymers, polyamides, polyurethane polymers, polyimides, metals, and the like.

Figure 1 describes an embodiment in which the arms 3 are deformed and expanded outward in the radial direction by the sliding operation of the shaft 4 and the tubular member 2. However, the arms 3 can be expanded outward in the radial direction without the shaft 4 by using the arms 3 made of, for example, a shape-memory alloy or a shape-memory polymer which deforms with heat, or of a bimetal in which two metal plates having different coefficients of thermal expansion are bonded together. Examples of the shape-memory alloy or shape-memory polymer include, but are not limited to, nickel titanium alloys and polyurethane polymers. Further, the arms 3 may be molded in an expanded state in advance, and the esophageal liquid-supply catheter 1 may be introduced into the body in another tube. Thus, when the arms 3 protrude from the distal end of the tube, the arms 3 can be deformed and expanded outward in the radial direction.

Examples of the shape of the esophageal liquid-supply catheter 1 on the handle side include, but are not limited to, a Y-shaped connector 6 having a valve structure 7, which allows the tubular member 2 and the shaft 4 to be slid and fixed.

The handle side of the esophageal liquid-supply catheter 1 is connected to an extension tube 10 which is capable of delivering and sucking a liquid into the lumen of the tubular member 2. When a liquid is delivered from a syringe 11 connected to the extension tube 10, the liquid passes through a space (gap) between the inner wall of the tubular member 2 and the outer wall of the shaft 4, and then discharged from a fluid hole 13 formed by the gap between the distal end of the tubular member 2 and the shaft 4. The liquid delivered to the esophagus is preferably a temperature-controlled liquid. For example, when used in combination with an ablation catheter which is heated, the temperature is preferably 5°C or lower. When used in combination with an ablation catheter which is cooled, the temperature is preferably 25°C or higher and 40°C or lower. In addition to the syringe 11, a known device used for an esophageal liquid-supply catheter can be used as a device for delivering and sucking a liquid to the esophageal liquid-supply catheter 1, which is provided outside the esophageal liquid-supply catheter 1.

One or more temperature sensors 8 are arranged on the shaft 4. Lead wire(s) of the temperature sensor(s) (not shown) extend inside the shaft 4 or inside the lumen of the tubular member 2 to the rear end of the esophageal liquid-supply catheter 1, from which the lead wire(s) are connected to a connector 9 arranged on the handle side. The connector 9 is connected to a temperature display device, and thus, it is possible to measure the temperature inside the esophagus.

Figure 3 shows the esophageal liquid-supply catheter 1 according to the second embodiment of the present invention. That is, one or more temperature sensors 8 are arranged on the arms 3. In this case, the lead wires of the temperature sensors extend inside the arms 3 or inside the lumen of the tubular member 2 to the rear end of the esophageal liquid-supply catheter 1, from which the lead wires are connected to a connector 9 arranged on the handle side. One or more of the temperature sensors may be arranged on both the shaft 4 and the arms 3.

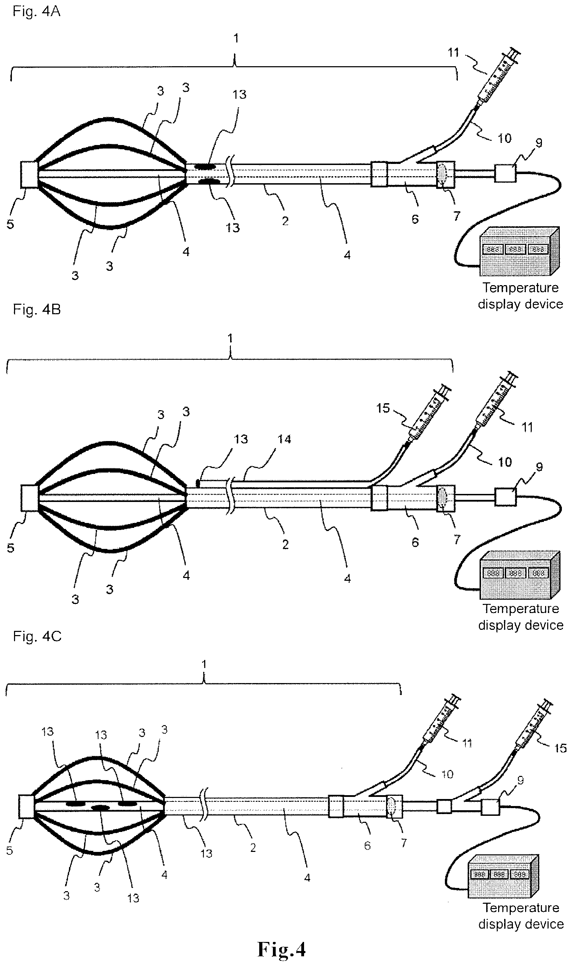

Figure 4 shows the esophageal liquid-supply catheter 1 according to the third embodiment of the present invention. In Figure 4A, fluid holes 13 are arranged on the tubular member 2.

In Figure 4B, the fluid hole 13 is at the distal end of a fluid tube 14 that is provided along the tubular member 2. In this case, a syringe 15 is connected to the rear end side of the fluid tube 14.

In Figure 4C, the fluid holes 13 are arranged on the shaft 4. In this case, the shaft 4 is a tube (catheter) having a lumen, and the syringe 15 is connected to the proximal end side of the lumen. In this embodiment, the liquid is flowed from the syringe 15 to the lumen of the shaft 4 in the form of a catheter.

As described above, the fluid hole(s) 13 may be arranged anywhere on the esophageal liquid-supply catheter 1, and may be a combination of Figures 1, 4A, 4B, and 4C. Further, any of the fluid holes 13 can be used not only for delivering the liquid but also for sucking the liquid, for example, for sucking the liquid which has been excessively delivered. Moreover, the syringe 11 and the syringe 15 can be used together.

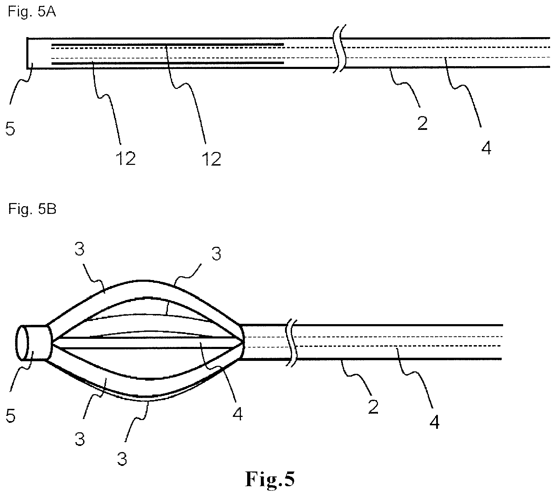

Figures 5A and 5B show the esophageal liquid-supply catheter 1 according to the fourth embodiment of the present invention. That is, by inserting two or more slits 12 into the tubular member 2, the arms 3 are formed by the tubular member present between the slits. Figure 5A shows the state in which the arms 3 are closed, and Figure 5B shows the state in which the arms 3 are expanded outward in the radial direction.

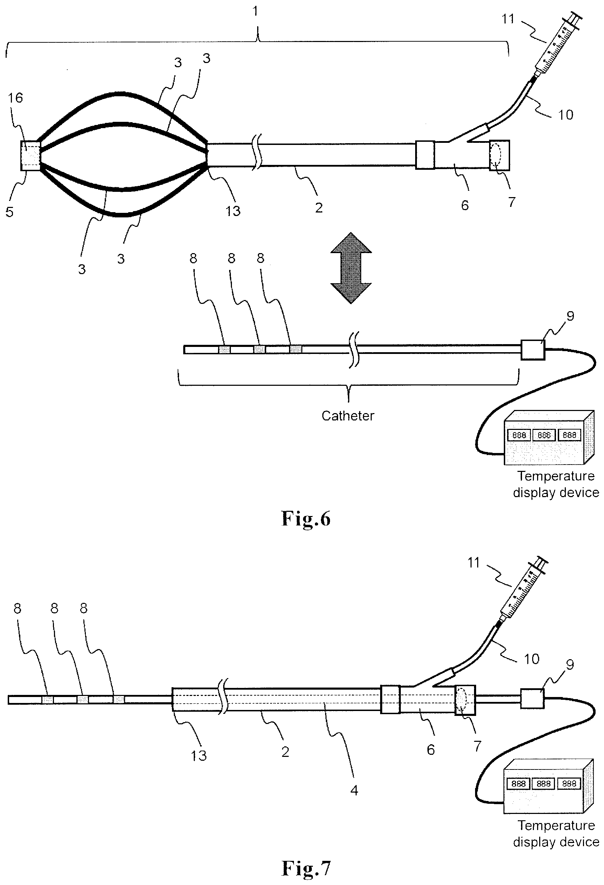

Figure 6 shows the esophageal liquid-supply catheter 1 according to the fifth embodiment of the present invention. That is, the distal end tip 5 has an attachment mechanism 16 that can be fitted and detached from the distal end of the shaft 4, and thus the shaft 4 can be replaced with various catheters depending on the application.

The attachment mechanism 16 is, for example, a columnar silicon rubber having a slit or a hole of about 5 Fr (French catheter scale) = 1.667 mm. By fixing the attachment mechanism 16 to the distal end tip 5, various catheters including commercially available products can be used as a shaft 4 and fitted and detached from the distal end tip 5. The attachment mechanism 16 is not limited to this and may be any mechanism as long as it can fix various catheters to the distal end tip 5. Further, a catheter having a function for measuring the temperature and having a diameter of 1.667 mm to 5 mm is preferably used as a shaft because the temperature inside the esophagus can be measured.

As described above, in the present invention, a commercially available catheter can be used as a shaft. When the above-mentioned attachment mechanism 16 is fixed to the distal end tip 5, the user (doctor) can use any desired catheter as the shaft and assemble the esophageal liquid-supply catheter of the present invention before use. That is, the above-mentioned esophageal liquid-supply catheter of the present invention excluding the shaft can be used as an intermediate component for manufacturing the esophageal liquid-supply catheter of the present invention, and thus, the present invention provides such an intermediate component as well.

### EXAMPLES

The specific examples of the esophageal liquid-supply catheter of the present invention will be described below. The term "length" refers to the length in the longitudinal direction.

### (Example 1)

A polyvinyl chloride tube having an outer diameter of 4.7 mm, an inner diameter of 3.8 mm, and a length of 550 mm was formed to use as a tubular member 2.

A polyurethane tube having an outer diameter of 2.3 mm, an inner diameter of 1.2 mm, and a length of 580 mm was formed to use as a shaft 4. A copper wire with a diameter of 0.1 mm and a length of 650 mm coated with an electrically insulating polytetrafluoroethylene protective coating and a constantan wire with a diameter of 0.1 mm and a length of 650 mm coated with an electrically insulating polytetrafluoroethylene protective coating were welded together only at the tips of the two metal wires to form a thermocouple. This welded thermocouple was fixed by soldering inside a SUS304 pipe having an outer diameter of 2.5 mm, an inner diameter of 2.3 mm, and a length of 4.4 mm. Thus, a temperature sensor 8 was formed. Three temperature sensors 8 were fixed by swaging at positions of 30 mm, 40 mm, and 50 mm from the distal end side of the shaft 4, and the copper wires and the constantan wires were all connected to the metal pins of the connector 9 via the lumen of the shaft 4.

A Y-shaped connector 6 was fixed to the rear end of the tubular member 2 with an adhesive, and the shaft 4 was inserted from the rear end side of the Y-shaped connector 6 to form a double-tube shaft. The Y-shaped connector 6 has a valve structure 7, and thus, the shaft 4 can be slid, and liquid can be prevented from leaking from the gap between the tubular member 2 and the shaft 4. An extension tube 10 having a length of 20 mm and a syringe 11 having a capacity of 30 mL were connected to the Y-shaped connector 6 so that a liquid could be delivered into the tubular member 2.

A polyvinyl chloride tube having an outer diameter of 4.7 mm, an inner diameter of 3.8 mm, and a length of 20 mm was formed to use as a distal end tip 5. A silicon tube having an outer diameter of 3.8 mm, an inner diameter of 1.7 mm, and a length of 20 mm was used as an attachment mechanism 16 and fixed inside the distal end tip 5 with an adhesive. The distal end side of the shaft 4 was inserted into the attachment mechanism 16, and thus, the distal end tip 5 and the shaft 4 were fixed.

Four vinyl chloride flat plates, each having a width of 4.5 mm, a thickness of 0.5 mm, and a length of 50 mm, were formed as arms 3. That is, the maximum width of the cross section of the arms 3 was 4.5 mm. The four arms 3 were heat-welded and fixed to the tubular member 2 and the distal end tip 5 such that the angle of each with the central axis of the shaft 4 was 90° (that is, at equal intervals around the shaft 4). An esophageal liquid-supply catheter of Example 1 (hereinafter. "Example 1") was thus prepared.

### (Example 2)

An esophageal liquid-supply catheter of Example 2 (hereinafter, "Example 2") was prepared in the same manner as in Example 1 except that the width of the vinyl chloride flat plates used as the arms 3 was changed to 0.9 mm. The maximum width of the cross section of the arms 3 was 1.0 mm,

### (Comparative Example 1)

Figure 7 is a schematic view of an esophageal liquid-supply catheter of Comparative Example 1. The attachment mechanism 16, the distal end tip 5 and the arms 3 were removed from the esophageal liquid-supply catheter of Example 1, and distal end of the shaft 4 was sealed with an adhesive. Thus, an esophageal liquid-supply catheter of Comparative Example 1 (hereinafter. "Comparative Example 1") was prepared. As shown in the figure, in Comparative Example 1, the arms 3 are removed, and there is no member that expands outward in the radial direction.

### (Example 3)

An esophageal liquid-supply catheter of Example 3 (hereinafter, "Example 3") was prepared in the same manner as in Example 1 except that the width of the vinyl chloride flat plates used as the arms 3 was changed to 0.5 mm. The maximum width of the cross section of the arms 3 was 0.7 mm.

The method and results of the comparative evaluation of Example 1, Example 2, Comparative Example 1, and Example 3 will now be described.

### (Reduction Effect of Temperature Transmission)

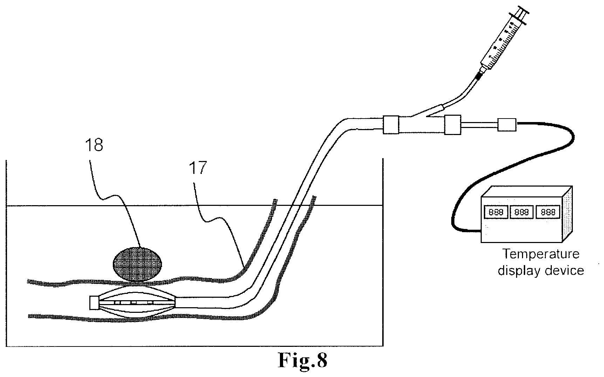

Figure 8 is a schematic view illustrating an evaluation system for confirming the reduction effect of the temperature transmission by an esophageal liquid-supply catheter. In this evaluation, an ablation catheter with a balloon which can be heated in the atrium was assumed. Hot water at 70°C was circulated at a rate of 1 L/min in a polyurethane balloon having a thickness of 30 µm and a diameter of 30 mm, and used as a heat source 18. Due to the circulation of the hot water, the surface temperature of the balloon is maintained at about 65°C in warm water at 37°C. A pig esophagus 17 into which Example 1, Example 2, Comparative Example 1, or Example 3 was inserted and the heat source 18 were immersed in the warm water at 37°C.

The shaft 4 of Example 1, Example 2 and Example 3 was slid by 20 mm toward the proximal end side relative to the tubular member 2. In this case, a force in the longitudinal direction was applied to the arms 3. and the arms 3 were deformed and expanded outward in the radial direction. The esophageal temperature was measured with the temperature sensors 8 of Example 1, Example 2, Comparative Example 1 and Example 3. When the esophageal temperature reached 40°C, a liquid having a temperature of 5°C was delivered at the delivery amount of 10 mL/time by the syringe 11 at a rate of 10 mL/sec.

In order to confirm the reduction effect of the temperature transmission when the esophagus was compressed from the outside, the heat source 18 was pressed against the pig esophagus 17 with a load of 0N, 3N and 5N.

The liquid delivery operation as mentioned above was carried out three times using Example 1, Example 2, Comparative Example 1 and Example 3.

In any of the above cases, the esophageal temperature measured by the temperature sensors 8 was about 50°C when the liquid was not delivered. Table 1 shows the measurement results (numerical values are in °C) of the decrease in the esophageal temperature due to the liquid delivery in each Example.

**[Table 1]**

| Example | Load | 0N | 3N | 5N |
|---|---|---|---|---|
| Example 1 | First time | -27.5 | -26.4 | -14.7 |
| | Second time | -28.8 | -25.0 | -15.9 |
| | Third time | -29.3 | -25.6 | -14.5 |
| | Mean | -28.5 | -25.7 | -15.0 |
| Example 2 | First time | -28.3 | -25.2 | -14.1 |
| | Second time | -27.4 | -24.7 | -14.9 |
| | Third time | -28.0 | -24.9 | -15.4 |
| | Mean | -27.9 | -24.9 | -14.8 |
| Comparative example 1 | First time | -23.1 | -10.7 | 0 |
| | Second time | -22.0 | -9.1 | 0 |
| | Third time | -22.6 | -10.2. | 0 |
| | Mean | -22.6 | -10.0 | 0 |
| Example 3 | First time | -26.9 | -20.7 | -10.1 |
| | Second time | -27.2 | -19.3 | -9.5 |
| | Third time | -26.5 | -18.2 | -9.0 |
| | Mean | -26.9 | -19.4 | -9.5 |

The comparison of Example 1, Example 2 and Comparative Example 1 confirmed that, in Comparative Example 1, the esophageal temperature decreased at a load of 0N and a load of 3N but not at a load of 5N because the liquid was no longer able to be delivered, and that in Examples 1 and 2, the esophageal temperature decreased by about 15°C even at a load of 5 N.

That is, when a liquid is delivered to the esophagus using a tubular member 2 not having arms 3, the liquid cannot be delivered efficiently if the esophagus is pressed and compressed from the outside due to the patient's condition, the weight of an organ, or the like. However, Example 1 and Example 2 demonstrated that the liquid could be efficiently delivered because the esophagus was supported from the inside.

The comparison of Example 1, Example 2 and Example 3 showed that the esophageal temperature decreased even at a load of 5 N in Example 3, but the decrease in the esophageal temperature was smaller than in Example 1 and Example 2.

The maximum width of the cross section of the arms 3 of Example 1 is 4.5 mm, the maximum width of the cross section of the arms 3 of Example 2 is 1.0 mm, and the maximum width of the cross section of the arms 3 of Example 3 is 0.7 mm. That is, it was confirmed that the maximum width of the cross section of the arms 3 is preferably 1.0 mm or more in order to efficiently deliver the liquid. In Examples 1, 2 and 3, the polyvinyl chloride arms 3 which did not have the sufficient rigidity for the expansion of the pig esophagus 17 in the direction of the heat source 18 were used. However, as long as the maximum width of the cross section of the arms 3 was 1.0 mm or more, particularly efficient liquid delivery was possible. It is clear that the arms 3 with higher rigidity may be used in consideration of the patient's condition, the weight of an organ, and the like.

### INDUSTRIAL APPLICABILITY

The present invention can be used as an esophageal liquid-supply catheter that can be used in combination with catheter ablation for treating arrhythmias such as atrial fibrillation.

### DESCRIPTION OF THE REFERENCE NUMERALS

- 1: Esophageal liquid-supply catheter
- 2: Tubular member
- 3: Arms
- 4: Shaft
- 5: Distal end tip
- 6: Y-shaped connector
- 7: Valve structure
- 8: Temperature sensor
- 9: Connector
- 10: Extension tube
- 11: Syringe
- 12: Slits
- 13: Fluid hole
- 14: Fluid tube
- 15: Syringe
- 16: Attachment mechanism
- 17: Pig esophagus
- 18: Heat source

## Claims

1. An esophageal liquid-supply catheter, comprising a tubular member, a plurality of arms with the proximal ends fixed to the distal end of said tubular member, and a distal end tip fixed to the distal ends of said arms, wherein said plurality of arms can be deformed and expanded outward in the radial direction of said tubular member.

2. The esophageal liquid-supply catheter according to claim 1, comprising a shaft which is inserted through said tubular member and which is fixed on the distal end to said distal end tip, wherein said arms are expanded outward in the radial direction when said shaft is slid toward the proximal end relative to said tubular member.

3. The esophageal liquid-supply catheter according to claim 2, wherein a space exists between the inner wall of said tubular member and the outer wall of said shaft, and a liquid is flowed in said space.

4. The esophageal liquid-supply catheter according to claim 1 or 2, further comprising a liquid-supply tube on the outer wall of said tubular member.

5. The esophageal liquid-supply catheter according to claim 2, wherein said shaft is in the form of a catheter, and a liquid to be delivered is flowed through said shaft.

6. The esophageal liquid-supply catheter according to any one of claims 1 to 5, wherein said arms or said shaft has a temperature sensor.

7. The esophageal liquid-slipply catheter according to any one of claims 1 to 6, wherein said tubular member has two or more slits, and said arms are constituted by said tubular member present between said slits.

8. The esophageal liquid-supply catheter according to any one of claims 1 to 7, wherein the cross section of said arms has a maximum width of 1 mm or more.

9. The esophageal liquid-supply catheter according to any one of claims 2 to 8, wherein said distal end tip and the distal end of said shaft can be fitted with and separated from each other.

10. The esophageal liquid-supply catheter according to any one of claims 2. 5 to 9. wherein said shaft is a catheter having a diameter of 1.667 mm to 5 mm.

11. An intermediate component for producing the esophageal liquid-supply catheter according to any one of claims 2 to 9, wherein said shaft is excluded from said esophageal liquid-supply catheter comprising said shaft according to any one of claims 2 to 9.
